Europäisches Patentamt

European Patent Office

Office européen des brevets

(11) Numéro de publication: **0 375 555**
**A1**

## DEMANDE DE BREVET EUROPEEN

(21) Numéro de dépôt: 89403601.1

(22) Date de dépôt: 21.12.89

(51) Int. Cl.⁵: **C07K 7/06, C07K 7/08, G01N 33/569, A61K 39/42, C12P 21/00**

(30) Priorité: 23.12.88 FR 8817097

(43) Date de publication de la demande:
27.06.90 Bulletin 90/26

(84) Etats contractants désignés:
AT BE CH DE ES FR GB GR IT LI LU NL SE

(71) Demandeur: MEDGENIX GROUP, S.A.
1, place d'Italie
B-4000 Liège(BE)

(72) Inventeur: De Martynoff, Guy
Rue des Pêcheries, 69
B-1170 Bruxelles(BE)
Inventeur: Brasseur, Robert
250, Voies des Gérons
B-5351 Haillot(BE)
Inventeur: Zeicher, Marc
Rue Danse, 18
B-1180 Bruxelles(BE)

(74) Mandataire: Warcoin, Jacques et al
Cabinet Régimbeau 26, avenue Kléber
F-75116 Paris(FR)

(54) Peptides, anticorps dirigés contre ces peptides, et procédés de détection et de dosage de papillomavirus HPV16.

(57) La présente invention concerne des peptides contenant au moins un déterminant antigénique des protéines E6, E7, L1 ou L2 des papillomavirus humains, déterminant spécifique du papillomavirus humain de type 16 : HPV16.

Ces peptides selon l'invention sont utiles principalement dans des sérodiagnostics pour détecter et doser des anticorps spécifiques du papillomavirus du HPV16 dans des échantillons biologiques.

Ces peptides couplés à une molécule support présentant des propriétés immunogéniques sont également utilisés pour produire des anticorps dirigés contre ces épitopes afin notamment de détecter par des techniques immunohistochimiques à l'aide de ces anticorps les cellules infectées par le virus HPV16.

## PEPTIDES, ANTICORPS DIRIGES CONTRE CES PEPTIDES, ET PROCEDES DE DETECTION ET DE DOSAGE DE PAPILLOMAVIRUS HPV16.

La présente invention concerne des peptides contenant au moins un déterminant antigénique des protéines E6, E7, L1 ou L2 du papillomavirus humain de type 16 : HPV16. Ces déterminants sont spécifiques du virus HPV16.

La présente invention concerne plus précisément des peptides notamment de 7 à 20 aminoacides, obtenus à partir de, c'est-à-dire comprenant en totalité ou en partie, une des dix séquences peptidiques de 9 à 14 aminoacides sélectionnées parmi les meilleurs épitopes et les plus spécifiques des quatre protéines mentionnées de HPV16 qui seront explicitées ci-après.

Ces peptides selon l'invention ont été sélectionnés de façon originale en vue principalement d'une utilisation dans des sérodiagnostics pour détecter et doser des anticorps spécifiques du papillomavirus HPV16 dans des échantillons biologiques, ce qui n'avait jamais pu être réalisé jusqu'à présent.

Mais, ces peptides couplés à une molécule support présentant des propriétés immunogéniques, c'est-à-dire permettant une immunisation plus efficace contre les peptides, peuvent également être utilisés pour produire des anticorps dirigés contre ces épitopes afin notamment de détecter par des techniques immunohistochimiques à l'aide de ces anticorps les cellules infectées par le virus HPV16.

La présente invention concerne donc aussi, d'une manière générale, des procédés de détection et/ou dosage du virus HPV16 dans des échantillons biologiques.

Et en particulier, la présente invention concerne un procédé de détection et/ou de dosage d'anticorps spécifiques du virus HPV16 dans des sérums humains et un procédé de détection de protéines virales de HPV16 par des techniques immunohistochimiques.

L'invention concerne également lesdits peptides couplés à un élément de marquage. On entend dans la présente demande par "élément de marquage" une substance directement détectable telle qu'une substance radioactive, une substance fluorescente, ou encore un enzyme et ceci de manière non limitative, ou une substance indirectement détectable, par exemple la biotine qui n'est pas détectable en tant que tel, mais via l'avidine ou la streptavidine, ces dernières étant liées à une substance directement détectable, telle qu'une substance radioactive, une enzyme ou encore une substance fluorescente.

La présente invention concerne également les anticorps obtenus à partir des peptides selon l'invention rendus immunogéniques par couplage à une protéine porteuse immunogénique via un lien chimique covalent.

La présente invention concerne donc également des conjugués des peptides selon l'invention couplés à une macromolécule support présentant, si cela est souhaité, des propriétés immunogéniques. Les propriétés immunogéniques étant souhaitées, le conjugué est destiné à préparer des anticorps. En revanche, lorsque le conjugué est destiné à faciliter l'adsorption du peptide sur une phase solide dans le cadre d'un procédé de détection et de dosage sur phase solide, on cherchera à utiliser une molécule support présentant peu ou pas de propriétés immunogéniques.

L'intérêt de l'invention dans ses différents objets est lié à l'importance croissante attribuée au virus HPV16 dans les cancers du col utérin.

De tous les cancers humains liés à des infections virales, celui du col utérin est de loin le plus fréquent - près de 16 % des cancers chez la femme - avec une incidence de 500 000 femmes par an, essentiellement dans les pays en voie de développement (Marx, 1986). Bien qu'il ait décliné en incidence et en mortalité grâce à des programmes de détection précoce, ce cancer représente toujours un problème de santé publique important dans les pays d'Europe et d'Amérique du Nord de par l'augmentation de la fréquence et de la précocité d'apparition des lésions dysplasiques (précancéreuses). L'incidence de ces lésions varie de 3 à 5 pour mille par an selon les régions, avec une augmentation annuelle de 10 à 15 %.

Depuis plus de vingt ans, des études épidémiologiques ont révélé un lien existant entre les dysplasies génitales (col utérin et vagin) et un facteur cancérogène à longue latence, transmis par voie sexuelle. De nombreux agents infectieux furent incriminés comme facteurs étiologiques (dont le virus herpès simplex - HSV) mais ce n'est que récemment, sur base de données cytologiques et d'expériences d'hybridation moléculaire, que quelques types spécifiques de papillomavirus humains (HPV) ont été associés à plus de 90 % des lésions prémalignes et cancéreuses du tractus génital (Ostrow et coll, 1987 ; zur Hausen, 1987).

Cette situation résulte d'une remarquable spécificité de ces virus pour l'hôte et le tissu qu'ils infectent et de l'absence, même encore actuellement, de système de propagation virale en culture cellulaire in vitro.

Essentiellement épithéliotropes, les papillomavirus ont leur cycle réplicatif synchronisé avec la différenciation des épithéliums squameux stratifiés et seules les couches superficielles - kératinocytes différenciés et cornéocytes - contiennent des virus infectieux.

Cette dépendance envers la différentiation épithéliale explique l'impossibilité actuelle de produire des particules virales in vitro et l'absence d'antigènes disponibles pour différencier sérologiquement ces virus.

L'introduction des techniques de clonage et l'application de procédures d'hybridation moléculaire ont permis de contourner ces difficultés et de caractériser une cinquantaine de papillomavirus humains impliqués dans diverses lésions (revue : Mac Cance, 1986 ; Pfister, 1987).

L'organisation structurale, remarquablement bien conservée entre eux, indique que leur génome viral est divisé en deux régions : la région précoce contenant les phases de lecture ouvertes (ORF) E1 à E8 - qui coderaient pour les fonctions de réplication épisomale, de contrôle transcriptionnel et de transformation - et la région tardive dont les deux ORF (L1 et L2) produiraient les protéines de capside. Ces gènes tardifs, qui ne sont exprimés qu'aux stades avancés de la différenciation cellulaire, ne sont jamais exprimés en culture de kératinocytes in vitro (Pilacinski et coll, 1987).

L'infection à papillomavirus apparaît comme l'événement néoplasique le plus fréquent survenant dans l'épithélium cervical des jeunes femmes, souvent rapidement (1 à 2 ans) après le début de l'activité sexuelle.

Les types d'HPV les plus fréquemment retrouvés dans ces lésions sont les HPV16 et 18 avec une fréquence respective de 50 % (35 à 68 %) et 15 % (10 à 25 %).

Si, approximativement, un quart des lésions régressent, la moitié persistent et un quart progressent (Syrjanen et coll, 1985).

La capacité tumorale d'HPV16 a été étayée récemment par la transformation morphologique de lignées cellulaires établies de fibroblastes de souris et de rats (Yasumoto et coll, 1986 ; Kanda et coll, 1987 ; Noda et coll, 1988) ainsi que celle de fibroblastes et de kératinocytes humains (Pirisi et coll, 1987). Les analyses transcriptionnelles des lignées cellulaires contenant le provirus HPV16 révèlent l'expression prédominante des séquences E6-E7 (Schwarz et coll, 1985 ; Baker et coll, 1987) et l'emploi d'anticorps dirigés contre les protéines E6 ou E7 - exprimées par des vecteurs bactériens ou eucaryotiques - confirme la présence des produits de ces deux gènes dans les lignées de carcinome cervical (Seedorf et coll, 1987 ; Smotkin et coll, 1987 ; Androphy et coll, 1987 ; Mallon et coll, 1987).

Toutes ces analyses indiquent que, contrairement aux autres gènes viraux, les gènes E6 et E7 sont exprimés dans la vaste majorité des lésions prémalignes et cancéreuses associées à l'infection par HPV16.

A cause du manque d'antigènes spécifiques aux HPV, le rôle des mécanismes immunitaires humoral et cellulaire contre ces virus est bien moins connu chez l'être humain. La plupart des données sur les réponses immunitaires aux HPV sont obtenues avec des mélanges de ces virus utilisés comme antigènes (Baird, 1983).

Les rares études utilisant des antigènes spécifiques montrent l'importance de la protéine structurale majeure de la capside virale (L1) dans les réactions antigéniques.

Il convient de souligner que si des peptides du virus HPV16 ont pu être décrits dans l'état de la technique, aucun n'ont permis d'obtenir jusqu'à présent des tests de détection spécifique du virus HPV16 qui permettent de conclure avec certitude à l'infection ou non par ledit virus, notamment dans le cadre de sérodiagnostics.

On peut citer en particulier un abrégé récent d'une communication présentée en Mai 1988 au "7ème International Papillomavirus Workshop" ("Synthetic peptides and anti-peptide antibodies in the study of HPV16 capsid proteins", Barbara K. Beiss et al.) dans lequel les auteurs reconnaissent n'avoir pu sélectionner aucun épitope permettant de mener à bien de façon concluante des tests de sérodiagnostics pour la détection d'anticorps spécifiques de HPV16.

Précédemment, dans la demande de brevet EP 257 754 était décrit un certain nombre de peptides constituant des épitopes de HPV16. Ces épitopes ont été sélectionnés pour des histodiagnostics, c'est-à-dire en vue de leur faculté de produire des anticorps, lesquels étaient utilisés dans des tests mettant en oeuvre des techniques immunohistochimiques pour la détection de protéines de HPV16. Ces épitopes qui n'ont pas été sélectionnés dans les protéines L1 et L2, et qui sont plus longs que ceux proposés selon la présente invention, n'ont pas été validés pour des test de sérodiagnostics de détection d'anticorps spécifiques de HPV16.

C'est pourquoi, un but de la présente invention était principalement de proposer les épitopes les plus caractéristiques du virus HPV16 dans la perspective de détecter et doser dans le sérum de femmes infectées la présence d'anticorps dirigés contre les antigènes des HPV16 impliqués dans les cancers du col utérin.

Un autre but était d'obtenir des anticorps polyclonaux et monoclonaux dirigés contre ces épitopes afin notamment de détecter par des procédés de détection immunohistochimiques les cellules infectées par HPV16 notamment dans les frottis et biopsies cervicales.

Un autre but était de proposer des procédés et moyens utiles dans ces procédés pour détecter et/ou

EP 0 375 555 A1

doser les anticorps et antigènes associés aux HPV16.

Une technique de sélection original a permis selon l'invention de sélectionner dix courtes séquences peptidiques de 9 à 14 aminoacides.

La présente invention a donc pour objet des peptides contenant au moins un déterminant antigénique spécifique du papillomavirus de type 16 (HPV16), caractérisés en ce qu'ils consistent en une séquence peptidique d'au moins 7 aminoacides comprenant en totalité ou en partie une des dix séquences peptidiques suivantes :

(1) R K L P Q L C T E L
(Arg-Lys-Leu-Pro-Gln-Leu-cys-Thr-Glu-Leu)
qui correspond à la position 17-26 de la protéine E6.

(2) E K Q R H L D K K Q
(Glu-Lys-Gln-Arg-Leu-Asp-Lys-Gln)
qui correspond à la position 121-130 de la protéine E6.

(3) A E P D R A H Y N I V T F C
(Alu-Glu-Pro-Asp-Arg-Alu-His-Tyr-Asn-Ile-Val-Thr-Phe-Cys)
qui correspond à la position 45-58 de la protéine E7.

(4) P I K K P N N N K I
(Pro-Ile-Lys-Lys-Pro-Asn-Asn-Asn-Lys-Ile)
qui correspond à la position 77-86 de la protéine L1.

(5) K H T P P A P K E D D
(Lys-His-Thr-Pro-Pro-Alu-Pro-Lys-Glu-Asp-Asp
qui correspond à la position 455-465 de la protéine L1.

(6) G K R K A T P T T
(Gly-Lys-Arg-Lys-Alu-Thr-Pro-Thr-Thr)
qui correspond à la position 509-517 de la protéine L1.

(7) T T A K R K K R K L
(Thr-Thr-Alu-Lys-Arg-Lys-Lys-Lys-Arg-Lys-Leu)
qui correspond à la position 522-531 de la protéine L1.

(8) M R H K R S A K R T K
(Met-Arg-His-Lys-Arg-Ser-Ala-Lys-Arg-Thr-Lys)
qui correspond à la position 1-11 de la protéine L2.

(9) N K Q T L R T R S G K
(Asn-Lys-Gln-Thr-Leu-Arg-Thr-Arg-Ser-Glu-Lys)
qui correspond à la position 309-318 de la protéine L2.

(10) Y Y M L R K R R K
(Tyr-Tyr-Met-Leu-Arg-Lys-Arg-Arg-Lys)
qui correspond à la position 452-460 de la protéine L2.

Avantageusement, ces peptides selon l'invention comportent de 7 à 20 aminoacides.

De préférence, on peut citer les séquence (1) à (10) complètes, ainsi que les trois séquences suivantes :
- A E P D R A H Y N tirée de la séquence (3),
- K H T P P A P K E tirée de la séquence (5),
- N K Q T L R T R tirée de la séquence (9).

De préférence encore, on utilisera les séquences (2), (3), (7) et (10).

Et parmi celles-ci, avantageusement la séquence (2) : E K Q R H L D K K Q.

Comme il sera explicité plus loin, à des fins de couplage, les peptides selon la présente invention pourront comporter une cystéine terminale supplémentaire.

La présente invention a également pour objet des procédés de détection et dosage d'anticorps spécifiques du papillomavirus humain de type 16 (HPVI6) dans des échantillons biologiques, dans lesquels on met en oeuvre à titre de réactif des substances contenant des peptides selon l'invention.

Selon l'invention, ces peptides peuvent être mis en oeuvre de diverses façons selon le type de procédé de détection et/ou dosage auquel on a recours. Il existe en effet de nombreuses techniques de détection et/ou dosage d'anticorps bien connues de l'homme de l'art, en particulier les dosages immunologiques sur phase solide. Pour une application dans un type de dosage immunologique sur phase solide, les peptides selon l'inventon peuvent être couplés à une molécule destinée à s'adsorber sur la phase solide telle que la paroi du récipient d'essai.

Cette macromolécule pourra être une protéine et devra alors avantageusement être telle que pas ou peu d'anticorps lui soient spécifiques dans les sérums testés. On utilisera par exemple la sérum albumine

4

de bovin (BSA) ou de préférence la sérum albumine humaine (HSA). La HSA étant présente à haute dose dans les sérums humains, ceux-ci ne comportent pas d'anticorps qui lui soient spécifiques.

Les peptides pourront être couplés, par exemple, à la BSA sur les groupes carboxyliques avec comme agent de couplage la carbodiimide l-3-(diméthylaminopropyl)3-éthyl carbodiimide-HCL (réf. : HOARE & KOSHLAND (1967), J. Biol. Chem. 242, 2447-2453).

On peut également réalisé un couplage au groupe amine ($NH_2$) de la protéine à l'aide de glutaraldéhyde (réf. KAGEN, A & GLICK, M. (1979) "Oxytocin", Methods of Hormone Radioimmunoassay (B.B. JAFFE & H.R. BERHMAN, editions) pages 328-329, Academic Press, New York).

On pourra également réalisé un couplage aux groupes phénoliques (Tyr, Trp, Phe) à l'aide de Bis-diazo benzidine (BDB) (réf. BASSIRI, R.M. DVORAK, J. et UTIGER, R.D. (1979) "Thyrotropin-Releasing Hormone", Methods of Hormone Radioimmunoassay pages 46-47, Academic loress, New York).

Pour conserver ses caractéristiques antigéniques, le peptide sera avantageusement couplé à une de ses extrémités.

Un procédé permettant d'assurer un couplage en terminaison du peptide consiste en l'addition d'une cystéine à l'extrémité amino-terminale du peptide pour le couplage à l'albumine sérique humaine, par l'intermédiaire du m-maléimidobenzoyl-N-hydroxysuccinimide (M.B.S.) (réf. : LERNER, R.A. et al. (1981) Proc. Natl. Acad. Sci. USA 79, 3403-3407 ; KITAGAWA, T., KAWASAKI, T., et MUNECHIKA, H. (1982) J. Biochem. 92, 585-590)..

La présente invention a donc également pour objet les peptides additionnés si nécessaire d'une cystéine terminale.

Ces conjugués de peptides et d'un molécule adsorbable sur phase solide seront utiles dans les procédés de dosage immunologique en phase solide du type immunométrique. Dans le cadre de ces dosages sur phase solide, le peptide peut également être lié à la phase solide, via un lien covalent.

Selon des dosages immunologiques en phase solide, objets de l'invention, une fois le peptide adsorbé ou lié de façon covalente à la phase solide, on ajoute l'échantillon contenant les anticorps à doser, puis soit un second anticorps couplé à un élément de marquage, second anticorps reconnaissant les anticorps à doser, soit un peptide selon l'invention également doté d'un élément de marquage. Après addition si nécessaire d'un élément détectable reconnaissant et se fixant à l'élément de marquage dans le cas où l'élément de marquage ne comporte pas lui-même un élément directement détectable, on procède au lavage de la phase solide et à la lecture du signal émis par l'élément détectable.

La présente invention a donc pour objet les conjugués d'un peptide selon l'invention avec une molécule susceptible de s'adsorber sur une phase solide utile dans un procédé de dosage sur phase solide conforme à l'invention.

Avantageusement, le peptide est couplé à une macromolécule, notamment une protéine contre laquelle peu ou pas d'anticorps sont présents dans les échantillons biologiques à doser, notamment, la sérum albumine de bovin ou la sérum albumine humaine.

Plus précisément, un procédé selon l'invention est caractérisé en ce que les conjugués des peptides et d'une molécule susceptible de s'adsorber sur phase solide sont adsorbés sur ladite phase solide, notamment sur la paroi d'un récipient d'essai avant incubation des sérums à doser, puis les anticorps spécifiques du papillomavirus HPV16 présents dans les sérums se fixent sur les peptides adsorbés et sont révélés par des seconds anticorps introduits dans le récipient d'essai qui reconnaissent et se fixent sur lesdits anticorps spécifiques de HPV16, lesdits seconds anticorps étant couplés à un élément de marquage, directement ou indirectement détectable.

Avantageusement, selon la présente invention, lesdits seconds anticorps sont des anticorps monoclonaux anti-IgG ou anti-IgM humains.

A titre de récipient d'essai, on peut utiliser des plaques de microtitration.

Selon la présente invention à titre d'élément de marquage sur lesdits seconds anticorps, on utilise une molécule directement détectable, telle qu'une molécule radioactive ou une molécule fluorescente ou encore une enzyme.

Toutefois, dans un mode de réalisation de la présente invention un élément de marquage consiste en une molécule indirectement détectable telle que la biotine, celle-ci étant détectée ultérieurement par l'adjonction d'une solution contenant de l'avidine ou de la streptavidine, ces dernières substances étant elles-mêmes dotées d'une molécule directement détectable, comme une enzyme dans le cadre de dosage du type ELISA ou une substance fluorescente telle que l'Europium dans le cadre d'un dosage du type DELFIA (réf. : FLUOROIMMUNOASSAYS AND IMMUNOFLUOROMETRIC ASSAYS - Clin. Chem. 31/3 - p. 359-370 (1985) - Ilkka Hemmilä ; EUROPIUM AS A LABEL IN TIME-RESOLVED IMMUNOFLUROMETRIC ASSAYS - Analytical Biochemistry 137 p. 335-343 (1984) - Ilkka Hemmilä et al.).

Les procédés de dosage mettant en oeuvre les techniques de type ELISA avec comme élément détectable une enzyme sont applicables pourvu qu'elles soient suffisamment sensibles. Les techniques de dosage radioimmunologique du type immunométrique comprenant notamment l'utilisation de l'iode 125 ou encore les techniques par fluorescence du type DELFIA comportant l'utilisation d'une molécule fluorescente comme molécule détectable sont suffisamment sensibles pour la détection d'anticorps spécifiques de l'HPV16 dans les sérums humains.

C'est pourquoi dans un mode de réalisation particulier de l'invention, on aura recours à un procédé de dosage immunologique du type DELFIA dans lequel lesdits seconds anticorps sont liés à de la biotine qui est ultérieurement reconnue par de la streptavidine couplée à un complexe d'Europium.

On peut citer plus précisément un procédé dans lequel :

- le peptide couplé à de la BSA ou de la HSA est adsorbé sur la phase solide consistant dans la paroi du récipient d'essai, telle qu'une plaque de microtitration, laquelle est ensuite lavée,
- les sites non spécifiques de la phase solide sont ensuite bloqués par une solution saturante,
- les sérums des patientes sont ensuite incubés dans le récipient ou sur les plaques, puis celles-ci sont lavées,
- des anticorps monoclonaux anti-IgG ou anti-IgM humains biotinylés sont incubés, puis la phase solide à nouveau lavée,
- le récipient lavé est ensuite incubé avec de la streptavidine marquée à l'europium,
- une solution permettant la libération des ions Europium et leur incorporation dans des édifices micellaires est ajoutée,
- et enfin, on effectue la lecture au fluoromètre.

La présente invention a donc également pour objet une trousse de dosage mettant en oeuvre un procédé selon l'invention, caractérisée en ce qu'elle comporte une phase solide sur laquelle sont adsorbés ou liés des peptides selon l'invention.

On peut citer une trousse de dosage mettant en oeuvre un procédé de dosage immunologique de type DELFIA, caractérisée en ce qu'elle comporte en outre des solutions d'anticorps monoclonaux anti-IgG ou anti-IgM humains biotinylés.

La présente invention a également pour objet les anticorps obtenus à partir des peptides selon l'invention, rendus immunogéniques par couplage à une protéine porteuse immunogénique, telle que la sérum albumine de bovin, la sérum albumine humaine, la gamma globuline bovine, des polypeptides tels que la polylysine, le copolymère de poly-L-lysine acides polyglutamiques, des glycoprotéines telles que les lipopolysaccharides, ou encore des protéines telles que l'hémocyanine de patelle (KLH) et la tyroglobuline porcine (Tg). Bien entendu, ces protéines immunogéniques sont citées à titre non limitatif.

Les anticorps, objet de la présente invention, peuvent être aussi bien des anticorps polyclonaux que monoclonaux obtenus par les techniques connues de l'homme de l'art.

La présente invention a donc également pour objet un procédé de détection de protéines virales spécifiques de HPV16 selon les techniques immunohistochimiques mettant en oeuvre des anticorps selon l'invention liés à un élément de marquage.

D'autres caractéristiques et avantages apparaîtront à la lumière des exemples qui vont suivre.

### EXEMPLE 1 : Détection par hybridation des papillomavirus humains dans différents types de lésions génitales

On a tout d'abord établi une collection de tissus (frottis et biopsies) et de sérums de patientes infectées par HPV16 ou HPV18 pour la mise au point ultérieure des histo- et sérodiagnostics.

La présence d'ADN viral a été testée sur des biopsies provenant d'environ quatre vingts patientes françaises ou maghrébines atteintes d'un carcinome épidermoïde invasif (n = 69) ou d'un adénocarcinome (n = 8) du col utérin.

Le grattage direct du tissu cancéreux sur filtre a été confirmé par dot blot sur l'ADN extrait d'une portion de la biopsie. Les sondes sont marquées par "multiprime DNA labelling" et hybridées en présence de lait dégraissé en poudre (milieu BLOTTO : 6 X SSC-Régilait 0,25 % ; Johnson et coll, 1984). Une analyse plus détaillée des tumeurs positives a été faite par "Southern blotting" pour vérifier la présence du virus sous forme épisomale ou intégrée dans les chromosomes, en copies uniques ou multimériques (transfert sur filtre nylon en présence d'acétate d'ammonium : Smith et Summer, 1980).

Contrairement à ce que suggéraient certaines études, nous avons détecté la présence d'HPV16 non intégré dans le génome cellulaire d'un petit nombre de patientes traitées pour un carcinome épidermoïde invasif (n = 4).

Au total, 48 % de ces patientes sont infectées par HPV16 (43 % de Maghrébines - 55 % de Françaises) et 15 % par HPV18 (14 % de Maghrébines - 16 % de Françaises). La nationalité, les paramètre familiaux (nombre d'enfants, ...), le dossier histopathologique (gravité des infiltrations inflammatoires, différenciation du stroma et taille, densité et nombre des cellules tumorales, ...) ainsi que la stade de la lésion selon la classification T.N.M. (Tumor Node Metastasis) n'ont révélé aucune différence statistiquement valable.

Seul l'âge des patientes semble indiquer une légère prédisposition à l'apparition du cancer chez les femmes infectées par HPV16.

## EXEMPLE 2 : Sélection de peptides synthétiques

Des algorithmes ont été utilisés pour prédire la structure secondaire de chaque protéine (Garnier et coll., 1978) et déterminer la formation d'hélices amphipatiques. Pour celles-ci, le moment hydrophobique moyen et l'hydrophobicité moyenne sont calculés selon la procédure d'Eisenberg et coll (1982, 1984) améliorée.

La procédure initiale d'Eisenberg permettait de déterminer, à partir de la séquence en acides aminés, les régions globulaires, de surface et, membranaires d'une protéine quelconque.

L'apport original de l'invention a été le perfectionnement de cette méthode d'analyse de manière à mettre en évidence de très courtes régions au sein de la protéine jouant le rôle de récepteurs vis-a-vis d'une autre protéine, à savoir le couple antigène-immunoglobuline.

Un segment de 7,9 et 11 acides aminés est déplacé le long de la séquence protéique et l'hydrophobicité moyenne ainsi que le moment hydrophobique moyen sont calculés par segment.

Ces deux paramètres sont ensuite placés comme une fonction de l'acide aminé central du segment, tout au long de la séquence.

Ces calculs utilisent l'échelle normalisée d'hydrophobicité d'Argos et coll (1982).

Dix peptides (de 9 à 14 aminoacides) sont ainsi sélectionnées dans quatre ORF virales (E6, E7, L1 et L2).

Pour estimer la spécificité de ces peptides, une recherche d'homologie a été menée par le programme de comparaisons de séquences peptidiques de Sellers et Goad, tel qu'il se trouve implémenté dans un logiciel.

Les comparaisons ont porté sur les protéines E1, E2, E6, E7, L1 et L2 des papillomavirus suivants : HPV1$_a$, HPV$_5$, HPV$_b$, HPV8, HPV11, HPV16, HPV18 et HPV33

Ce criblage a permis de sélectionner les dix séquences (9 à 14 aminoacides) spécifiques d'HPV16 suivantes :

(1) R K L P Q L C T E L
(Arg-Lys-Leu-Pro-Gln-Leu-Cys-Thr-Glu-Leu)
qui correspond à la position 17-26 de la protéine E6.

(2) E K Q R H L D K K Q
(Glu-Lys-Gln-Arg-Leu-Asp-Lys-Gln)
qui correspond à la position 121-130 de la protéine E6.

(3) A E P D R A H Y N I V T F C
(Alu-Glu-Pro-Asp-Arg-Alu-His-Tyr-Asn-Ile-Val-Thr-Phe-Cys)
qui correspond à la position 45-58 de la protéine E7.

(4) P I K K P N N N K I
(Pro-Ile-Lys-Lys-Pro-Asn-Asn-Asn-Lys-Ile)
qui correspond à la position 77-86 de la protéine L1.

(5) K H T P P A P K E D D
(Lys-His-Thr-Pro-Pro-Alu-Pro-Lys-Glu-Asp-Asp
qui correspond à la position 455-465 de la protéine L1.

(6) G K R K A T P T T
(Gly-Lys-Arg-Lys-Alu-Thr-Pro-Thr-Thr)
qui correspond à la position 509-517 de la protéine L1.

(7) T T A K R K K R K L
(Thr-Thr-Alu-Lys-Arg-Lys-Lys-Lys-Arg-Lys-Leu)
qui correspond à la positicn 522-531 de la protéine L1.

(8) M R H K R S A K R T K
(Met-Arg-His-Lys-Arg-Ser-Ala-Lys-Arg-Thr-Lys)
qui correspond à la position 1-11 de la protéine L2.

(9) N K Q T L R T R S G K

(Asn-Lys-Gln-Thr-Leu-Arg-Thr-Arg-Ser-Glu-Lys)

qui correspond à la position 309-318 de la protéine L2.

(10) Y Y M L R K R R K

(Tyr-Tyr-Met-Leu-Arg-Lys-Arg-Arg-Lys)

qui correspond à la position 452-460 de la protéine L2.

Ces peptides sélectionnés par ces algorithmes ont été synthétisés selon les méthodes maintenant bien connues de l'homme de l'art.

## EXEMPLE 3 : Mise au point de sérodiagnostics

Les peptides synthétiques (couplés à la BSA) sont testés par ELISA et DELFIA avec les anticorps dirigés contre eux avant d'être testés avec les sérums de patientes infectées par HPV16.

Les antigènes viraux sont adsorbés sur le plastique avant incubation des sérums dans les cupules. Les anticorps fixés sont révélés par des anticorps secondaires anti-lapin ou anti-souris de chèvre couplés à la peroxydase ou la biotine.

La technique de dosage immunologique non isotopique DELFIA (Dissociation Enhanced Lanthanide Fluoroimmunoassay) implique l'utilisation d'un anticorps ou de streptavidine lié(e) à un ion Europium ($Eu^{+3}$) avec des dérivés de l'EDTA.

La fluorescence des chélateurs lanthanides est mesurée par TR-FIA (Time-resolved fluoroimmunoassay) afin de profiter de la très haute sensibilité de cette procédure. Cette technique profite également des autres propriétés des chélateurs lanthanides comme la très grande différence de longueur d'onde entre leurs pics d'émission et d'absorption, leur bruit de fond fortement réduit en fluorescence et leur région d'excitation relativement large (Hemmilä et coll, 1984).

### A) Protocole DELFIA

1. Le peptide couplé à la BSA (10 μg/ml PBS) est adsorbé sur les puits de plaques à microfiltration toute la nuit à 4°C (50 μl/puits) (Titertek, Microstrips 12 Wells P.S. catalogue n° 78.591.99). Les plaques sont ensuite lavées 5 fois à l'aide de NaCL 9 ‰ - Tween 0,025 %.

2. Les sites non spécifiques sont ensuite bloqués par du PBS -FCS 20 % (FCS : sérum de veau foetal - décomplémenté) 2 h à température ambiante (200 μl/puits).

Les plaques sont ensuite lavées 5 fois à l'aide de NaCL 9 ‰ - Tween 0,025 %.

3. Les sérums des patientes, dilués 100 fois dans du PBS - FCS 20 % - Tween 0,1 %, sont incubés la nuit à 4°C (50 μl/puits).

Les plaques sont ensuite lavées 5 fois à l'aide de NaCl 9 ‰ - Tween 0,025 %.

4. Les anticorps monoclonaux de rat anti-IgG humains biotinylés (réf. LO-HG-22 : Experimental Immunololgy Unit, Clos Chapelle aux Champs, 30, 1200 BRUXELLES) sont dilués (400 ng/ml) dans du PBS - FCS 20 % - Tween 0,1 % avant d'être incubés 2 h à température ambiante (50 μl/puits).

Les plaques sont ensuite lavées 5 fois à l'aide de NaCl 9 ‰ - Tween 0,025 %.

5. Les puits sont ensuite incubés avec de la streptavidine -Europium (L.K.B. Wallac P.O. Box 10, 200101 Turku, FINLAND) (250 ng/ml ; 50 μl) pendant 30 min., lavés 5 fois puis mis en présence de l'"enhancement solution" (100 μl) pendant 5 min. (L.K.B. Wallac) (réf. 1244-104).

L'"enhancement solution" est une solution constituée par un tampon acétate-phtalate 0,1 M PH 3,2 de TritonX-100 I g/l, de 2-Naphthoyltrifluoroacétone (2-NTA) 15 μm, et d'oxide de Tri-n-octylphosphine (TOPO) 50 μm. Elle permet de libérer l'ion Europium ($Eu^{+3}$) lequel se trouve ensuite incorporé dans des chelates micellaires (Eu-(2-NTA)$_3$ (TOPO)$_{2-3}$) hautement fluorescents qui font l'objet d'une lecture au fluoromètre.

6. Lecture au fluoromètre "Arcus 1230" TR-FIA (L.K.B. Wallac).

L'emploi d'un anticorps monoclonal anti IgG humain permet d'augmenter la réponse spécifique et de diminuer le bruit de fond. On entend par "bruit de fond" la réponse d'anticorps qui se fixent de façon non spécifique sur les puits ainsi que d'anticorps qui reconnaissent et se fixent sur la BSA.

Les caractéristiques originales de ce protocole DELFIA et qui ont permis d'aboutir à un abaissement du bruit de fond sont également :

- le fait de bloquer les sites non spécifiques avec une solution de PBS et sérum de veau foetal décomplémenté au lieu d'utiliser de la BSA,

- le fait également de diluer les sérums de patientes dans une solution de PBS-FCS 20 % et additionnée de Tween, alors qu'en général les sérums de patientes sont dilués dans des solutions de BSA ou lait dégraissé.

B) Résultats

Le protocole ci-dessus a permis que le bruit de fond du signal dû à la protéine porteuse soit fortement réduit. Pour plus de 80 % des sérums (cancers ou normaux), il se situe actuellement à moins de 3000 coups par seconde (cps) comme il apparaît dans le tableau 1 ci-après (valeur en cps).

TABLEAU 1

|  | Enfants (n = 34) | Adultes normaux (n = 41） | Cancers du col utérin (n = 91) |
|---|---|---|---|
| Moyenne | 1.411 | 1.794 | 2.362 |
| Déviation Standard | 742 | 688 | 2.011 |

Les peptides sélectionnés ont été testés sur des sérums provenant :
- de patientes atteintes d'un cancer invasif du col utérin (n = 91) et dont on a pu déterminer, pour une portion d'entre elle, l'infection, ou non par HPV 16 par hybridation moléculaire sur l'ADN extrait de biopsies,
- d'adultes asymptomatiques (n = 41) dont nous connaissons la valeur en vitesse de sédimentation des protéines pour 30 de ces sérums,
- d'enfants très jeunes (1 à 5 ans ; n = 34) hospitalisés pour différentes affections. Il s'agit d'une population dont nous connaissons le dossier pathologique et que l'on peut considérer comme témoin négative dans la mesure où aucune contamination d'ordre sexuelle n'a put intervenir, ce qui est une condition pour l'infection par HPV16.

Des quatre peptides (2), (3), (7) et (10) comme référencés précédemment, testés sur plus de 220 sérums, le peptide (2) présente dans les conditions actuelles de pureté et de couplage la meilleure réponse spécifique à une infection par le virus HPV16 par rapport aux autres peptides. Ceci est vraisemblablement dû au fait que la structure du peptide (2) permet d'assurer que le couplage avec la BSA s'est fait en extrémité du peptide, préservant ainsi ses déterminants antigéniques, ce qui n'a sans doute pas été le cas pour les autres peptides, compte tenu du fait que le couplage a été réalisé selon la procédure classique en utilisant comme agent de couplage la carbodiimide.

La "cut off value", c'est-à-dire la valeur au delà de laquelle une présence d'anticorps spécifiques de HPV16 est significative, est estimée à 15 000 cps. En effet, cette valeur est conventionnellement assimilée à la valeur moyennne d'une population non infectée additionnée de trois fois la déviation standard, ce qui correspond en prenant la population d'enfants : moyen (3.265) + 3 x écart type (3945) = 15 101 cps).

La réponse spécifique au peptide (2) (signal total - "bruit de fond") exprimée en pourcentage de la population sélectionnée est indiquée dans le tableau 2 suivant :

| (cps) | a | b | c | d | e | f | g | h |
|---|---|---|---|---|---|---|---|---|
| Enfants | 82 | 6 | 12 | 0 | 0 | 0 | 0 | 0 |
| Adultes | 54 | 28 | 11 | 0 | 7 | 0 | 0 | 7 |
| Cancers | 28 | 21 | 19 | 9 | 8 | 13 | 3 | 33 |
| a : 0-5000 ; b : 5-10000 ; c : 10-15000 ; d : 15-20000 ; e : 20-25000 ; f : 25-50000 ; g :> 50000 ; h :> 15000. | | | | | | | | |

Il convient de remarquer qu'une valeur de 7 % de réponse spécifique pour une population d'adultes asymptomatique est très révélatrice de l'exactitude du test. Il est en effet généralement admis que cette valeur correspond à celle de sujets à risque dans une population asymptomatique, c'est-à-dire de sujets

9

EP 0 375 555 A1

infectés par HPV16 mais dont les lésions précancéreuses ne sont pas déclarées. Ce fait est corroboré par la valeur nulle obtenue entre 15-20000 cps.

D'autre part, la valeur de 33 % obtenue à partir d'une population de cancers du col utérin confirment les données admises selon lesquelles le virus HPV16 se trouve associé à environ 40 % des cancers du col utérin (réf. : Mc CANCE, D.J., CHESTER, P.M., CAMPION, M.J. et al. (1986) Br. Obster. Gynaecol. 92, 1101-1105 ; DURST, M., GISSMANN, L., IKENBERG, H. et zur Hausen, H. (1983) Proc. Natl. Acad. Sci. USA 80, 3812-3815).

Le signal spécifique au peptide n'est corrélé :
- ni à une valeur élevée de vitesse de sédimentation du sang des adultes asymptomatiques (donneurs de sang),
- ni à des symptômes cliniques particuliers chez les enfants : allergies, infections - virus EBV, streptocoques, oreillons, syndromes inflammatoires, etc).

En outre, et surtout pour les carcinomes utérins, une très bonne corrélation ( 80 %) apparaît entre les femmes infectées ou non par HPV16 par confirmation de la présence du virus dans les biopsies par Southern Blotting et le signal spécifiques au peptide (2).

## EXEMPLE 4 : Mise au point d'histodiagnostic

Les lapins sont immunisés contre les peptides synthétiques (couplés à la KLH) par trois injections intradermiques (500 mg de peptide).

Les anticorps polyclonaux de ces sérums sont testés par ELISA (les antisérums ont reconnu leur peptide couplé à la BSA).

Les souris sont immunisées contre les protéines virales par injections sous-cutanées ou péritonéales (100 mg de peptide).

Les anticorps monoclonaux dirigés contre les peptides synthétiques sont obtenus par la méthode décrite par Kölher et Milstein (1975). Ils sont testés ensuite par la méthode DELFIA.

## REFERENCES BIBLIOGRAPHIQUES

ANDROPHY, E.J., LOWY, D.R. et SCHILLER, J.T. (1987)
Nature 325, 70-73.
ARGOS, P., RAO, M.J.K. et HARGRAVE, P.A. (1982)
Eur. J. Biochem. 128, 565-575.
BAIRD, P.J. (1983)
Lancet, 17-18.
BAKER, C.C., PHELPS, W.C., LINDGREN, V., BRAUN, GONDA, M.A. et HOWLEY, P.M. (1987)
J. Virol. 61, 962-971.
EISENBERG, D., WEISS, R.M. et TERWILLIGER, T.C. (1982)
Nature 299, 371-374.
EISENBERG, D. (1984)
Ann. Rev. Biochem. 53, 595-623.
GARNIER, J., OSGUTHORPE, D.J.G. et ROBSON, B. (1978)
J. Mol. Biol. 120, 97-120.
HEMMILA, I., DAKUBU,S., MUKKALA, V-M., SIITARI, H. et LOVGRANT, T. (1984)
Analytical Bioch. 137, 335-343.
JOHNSON, D.A., GAUTSCH, J.W., SPROTSMAN, J.R. et ELDER, J.H. (1984)
Gene Analysis Techniques 1, 3-8.
KANDA, T., WATANABE, S. et YOSHIIKE, K. (1987)
Jpn. J. Cancer Res. 78, 103-108.
KOLER, G. et MILSTEIN, C. (1975)
Nature 256, 495-497.
LAEMMLI, V.K. (1970)
Nature 227, 680-685.
MAC CANCE, D.J. (1986)
Biochem. Biophys. Acta 823, 195-205.

MALLON, R.G., WOJCIECHOWICZ, D. et DEFENDI, V. (1987)
J. Virol. 61, 1655-1660.
MARX, J.L. (1986)
Science 231, 914-920
NODA, T., YAJIMA, H. et ITO, Y. (1988)
J. Virol. 62, 313-324.
OSTROW, R.S., MANIAS, D.A., CLARK, B.A., OKAGAKI, T., TWIGGS, L.B. et FARAS, A.J. (1987)
Cancer Res. 47, 649-653.
PFISTER, H. (1987)
Adv. Cancer Res. 48, 113-147.
PILACINSKI, W.P., GLASSMANN, D.L., KRZYZEK, R.A., JADOWSKI, P.L. et ROBINS, A.K. (1984)
Biotechnology 2, 356-360.
PIRISI, L., YASUMOTO, S., FELLER, M., DONIGER, J., DI PAOLO, J.A. (1987)
J. Virol. 61, 1630-1638.
SCHWARZ, E., FREESE, U.K., GISSMANN, L., MAYER, W., ROGGENBUCK, G., STREMLAN, A et zur HAUSEN (1985)
Nature 314, 111-114.
SEEDORF, K., OLTERSDORF, T., KRAMMER, G. et ROWEKAMP, W. (1987)
EMBO J. 6, 139-144.
SMITH, G.E. et SUMMERS, M.D. (1980)
Analytical Biochem. 109, 123-129.
SMOTKIN, D. et WETTSTEIN, F.O. (1987)
J.Viro. 61, 1686-1689.
SYRJANEN, K., PARKINEN, S., MANTYJARVI, R. VAYRYNEN, M. SYRJANEN, S., HOLOPAINEN, H., SAARIKOSKIS, S. et CASTREN, O. (1985)
Eur. J. Epidemiol. 1, 180-185.
TOWBIN, H., STAEHELIN, T. et GORDON, J. (1979)
Proc. Natl. Acad. Sci USA 76, 4350-4357.
TOWBIN, H. et GORDON, J. (1984)
Jour. Immun. Meth. 72, 313-340.
YASUMOTO, S., BURKHART, A.L., DONIGER, J. et DI PAOLO, J.A. (1986)
J. Virol. 57, 572-577.
Zur HAUSEN, H. (1987)
Cervical neoplasia Appl. Pathol. 5, 19-24.

## Revendications

1. Peptide contenant au moins un déterminant antigénique spécifique du papillomavirus de type 16 (HPV16), caractérisé en ce qu'il consiste en une séquence peptidique d'au moins 7 aminoacides comprenant en totalité ou en partie une des séquences peptidiques suivantes :

    (1) R K L P Q L C T E L
    (2) E K Q R H L D K K Q
    (3) A E P D R A H Y N I V T F C
    (4) P I K K P N N N K I
    (5) K H T P P A P K E D D
    (6) G K R K A T P T T
    (7) T T A K R K K R K L
    (8) M R H K R S A K R T K
    (9) N K Q T L R T R S G K
    (10) Y Y M L R K R R K

2. Peptide selon la revendication 1, caractérisé en ce qu'il comporte de 7 à 20 aminoacides.

3. Peptide selon l'une des revendications 1 et 2, caractérisé en ce qu'il consiste en une séquence peptidique choisie parmi une des séquences (1) à (10) complètes et les trois séquences

  - A E P D R A H Y N
  - K H T P P A P K E E
  - N K Q T L R T R

4. Peptide selon l'une des revendications 1 à 3, caractérisé en ce qu'il est choisi parmi les séquences :

(2) E K Q R H L D K K Q

(3) A E P D R A H Y N I V T F C

(7) T T A K R K K R K L

(10) Y Y M L R K R R K

5. Peptide selon la revendication 4, caractérisé en ce qu'il consiste en la séquence (2) E K Q R H L D K K Q .

6. Peptide selon l'une des revendications précédentes, caractérisé en ce qu'il est additionné si nécessaire d'une cystéine terminale.

7. Procédé de détection et dosage d'anticorps spécifiques du papillomavirus humain de type 16 (HPV16) dans des échantillons biologiques, caractérisé en ce qu'on utilise un réactif comportant un peptide selon l'une des revendications précédentes.

8. Conjugué d'un peptide selon l'une des revendications précédentes et d'une molécule susceptible de s'adsorber sur une phase solide utile dans un procédé de dosage en phase solide selon la revendication 7.

9. Conjugué selon la revendication 8, caractérisé en ce que le peptide est couplé à une macromolécule contre laquelle peu ou pas d'anticorps dans les échantillons biologiques à doser sont présents.

10. Conjugué selon la revendication 9, caractérisé en ce que le peptide est couplé à de la sérum albumine de bovin ou de la sérum albumine humaine.

11. Peptide selon l'une des revendications I à 7, caractérisé en ce qu'il est doté d'un élément de marquage utile notamment dans un procédé selon la revendication 7.

12. Procédé selon la revendication 7, caractérisé en ce que des conjugués selon l'une des revendications 8, 9 et 10 sont adsorbés sur une phase solide notamment sur la paroi d'un récipient d'essai avant incubation des sérums à doser, les anticorps spécifiques du papillomavirus HPV16 se fixant sur les peptides et étant révélés par des seconds anticorps introduits dans le récipient qui reconnaissent et se fixent sur lesdits anticorps spécifiques de HPV16, seconds anticorps couplés à un élément de marquage détectable.

13. Procédé selon la revendication 12, caractérisé en ce que l'élément de marquage est une molécule directement détectable telle qu'une molécule radioactive, une molécule fluorescente ou une enzyme, ou une molécule indirectement détectable telle que la biotine.

14. Procédé selon l'une des revendications 11 et 12, caractérisé en ce que lesdits seconds anticorps sont des anticorps monoclonaux anti-IgG ou anti-IgM humains.

15. Procédé selon l'une des revendications 12 à 14, caractérisé en ce qu'il consiste en un procédé de dosage immunologique du type DELFIA dans lequel lesdits seconds anticorps sont couplés à de la biotine et sont révélés après fixation sur les anticorps spécifiques de HPV16 liés aux peptides adsorbés sur phase solide, par de la streptavidine couplée à un complexe d'Europium.

16. Procédé selon la revendication 15, caractérisé en ce qu'il comporte les étapes suivantes :
- le peptide couplé à de la BSA ou de la HSA est adsorbé sur la phase solide consistant dans la paroi du récipient d'essai, laquelle est ensuite lavée,
- les sites non spécifiques de la phase solide sont ensuite bloqués par une solution saturante,
- les sérums des patientes sont ensuite incubés dans le récipient, puis celui-ci est lavé,
- des anticorps monoclonaux anti-IgG humains biotinylés sont à leur tour incubés, puis la phase solide à nouveau lavée,
- le récipient lavé est ensuite incubé avec de la streptavidine marquée à l'Europium,
- une solution favorisant la libération de l'Europium est ajoutée, et enfin
- on effectue la lecture au fluoromètre.

17. Trousse de dosage mettant en oeuvre un procédé selon l'une des revendications 7 et 12 à 16, caractérisée en ce qu'elle comporte une phase solide sur laquelle sont adsorbés ou liés des peptides selon l'invention.

18. Anticorps polyclonaux et anticorps monoclonaux obtenus à partir des peptides selon l'une des revendications 1 à 7, conjugués à une protéine porteuse immunogénique.

19. Procédé de détection de protéines virales spécifiques du virus HPV16 selon une technique immunohistochimique, caractérisé en ce qu'on utilise un anticorps selon la revendication 18, couplé à un élément de marquage consistant en une substance directement ou indirectement détectable.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,Y | EP-A-0 257 754 (STANFORD UNIVERSITY)<br>* En entier *<br>--- | 1-19 | C 07 K    7/06<br>C 07 K    7/08<br>G 01 N   33/569<br>A 61 K   39/42<br>C 12 P   21/00 |
| Y | WO-A-8 701 375 (INSTITUT PASTEUR et al.)<br>* En entier, en particulier revendications 1-3 *<br>--- | 1-19 | |
| Y | CHEMICAL ABSTRACTS, vol. 108, no. 13, 28 mars 1988, page 345, résumé no. 109354t, Columbus, Ohio, US; L. BANKS et al.: "Expression of human papillomavirus type 6 and type 16 capsid proteins in bacteria and their antigenic characterization ", & J. GEN. VIROL. 1987 68(12)<br>* Résumé *<br>--- | 1-19 | |
| A | CHEMICAL ABSTRACTS, vol. 109, no. 11, 12 septembre 1988, page 491, résumé no. 90524v, Columbus, Ohio, US; J.M. FIRZLAFF et al.: "Detection of human papillomavirus capsid antigens in various aquamous epithelial lesions using antibodies directed against the L1 and L2 open reading frames", & VIROLOGY 1988, 164(2), 467-77<br>* Résumé *<br>--- | 1-18 | **DOMAINES TECHNIQUES RECHERCHES (Int. Cl.5)**<br><br>C 07 K<br>A 61 K<br>G 01 N<br>C 12 P |
| A | CHEMICAL ABSTRACTS, vol. 109, no. 3, 18 juillet 1988, page 180, résumé no. 18185t, Columbus, Ohio, US; & DE-A-3 625 257 (BEHRINGWERKE AG) 04-02-1988<br>* Résumé *<br>---                          -/- | 1-18 | |

Le présent rapport a été établi pour toutes les revendications

| Lieu de la recherche | Date d'achèvement de la recherche | Examinateur |
|---|---|---|
| LA HAYE | 07-02-1990 | MASTURZO P. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
................................................................
& : membre de la même famille, document correspondant

EPO FORM 1503 03.82 (P0402)

Office européen
des brevets

**RAPPORT DE RECHERCHE EUROPEENNE**

Numero de la demande

EP 89 40 3601

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|-----------|---------------------------------------------------------------------------------|-------------------------|--------------------------------------|
| A | CHEMICAL ABSTRACTS, vol. 108, no. 1, 4 janvier 1988, page 151, résumé no. 1513e, Columbus, Ohio, US; J.M. FIRZLAFF et al.: "Polyclonal antibodies to human papillomavirus type-6b and type-16 bacterially derived fusion proteins", & CANCER CELLS 1987, 5 (PAPILLOMAVIRUSES) 105-13 <br> * Résumé * | 1-18 | |

DOMAINES TECHNIQUES
RECHERCHES (Int. Cl.5)

Le présent rapport a été établi pour toutes les revendications

| Lieu de la recherche | Date d'achèvement de la recherche | Examinateur |
|----------------------|-----------------------------------|-------------|
| LA HAYE | 07-02-1990 | MASTURZO P. |

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons
    ........................................................
& : membre de la même famille, document correspondant

EPO FORM 1503 03.82 (P0402)